(19) Europäisches Patentamt
European Patent Office
Office européen des brevets

(11) Veröffentlichungsnummer : **0 111 146**
**B1**

(12) # EUROPÄISCHE PATENTSCHRIFT

(45) Veröffentlichungstag der Patentschrift : **30.12.86**

(21) Anmeldenummer : **83110889.9**

(22) Anmeldetag : **02.11.83**

(51) Int. Cl.[4] : **C 07 D405/06**, C 07 D405/14, A 61 K 31/41, A 61 K 31/415// C07D407/06, C07D317/26, C07D407/04, C07D319/06

(54) **Antimykotische Hydroxyalkyl-azolyl-Derivate.**

(30) Priorität : **15.11.82 DE 3242236**

(43) Veröffentlichungstag der Anmeldung : **20.06.84 Patentblatt 84/25**

(45) Bekanntmachung des Hinweises auf die Patenterteilung : **30.12.86 Patentblatt 86/52**

(84) Benannte Vertragsstaaten : **BE CH DE FR GB IT LI**

(56) Entgegenhaltungen :
**EP-A- 0 043 419**
**EP-A- 0 062 236**
**EP-A- 0 078 594**
**EP-A- 0 085 842**
**EP-A- 0 085 843**
**Medicinal Dictionary 26A S. 93**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Krämer, Wolfgang, Dr.**
**Am Eckbusch 39/45**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Holmwood, Graham, Dr.**
**Krutscheider Weg 105**
**D-5600 Wuppertal 11 (DE)**
Erfinder : **Regel, Erik, Dipl.-Ing.**
**Untere Bergerheide 26**
**D-5600 Wuppertal 1 (DE)**
Erfinder : **Plempel, Manfred, Dr.**
**Zwengenberger Strasse 3c**
**D-5657 Haan (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 111 146 B1

Jouve, 18, rue St-Denis, 75001 Paris, France

**Beschreibung**

Die vorliegende Erfindung betrifft die Verwendung von neuen heterocyclisch substituierten Hydroxyalkyl-azolyl-Derivaten als antimikrobielle Mittel, insbesondere als Antimykotika.

Es ist bereits bekannt geworden, daß bestimmte 1-Hydroxyethyl-azolyl-Derivate gute antimykotische Wirkungen besitzen (siehe, z. B. EP-A-85 843, EP-A-85 842, EP-4-43 419). Auch die fungiziden Eigenschaften von Triazolylderivaten sink bekannt (siehe z. B. EP-A-62 236). Es erschien jedoch wünschenswert, Verbindungen mit noch besseren antimykotischen Eigenschaften aufzufinden.

Gefunden wurden die neuen heterocyclisch substituierten Hydroxyalkyl-azolyl-Derivate der allgemeinen Formel (I),

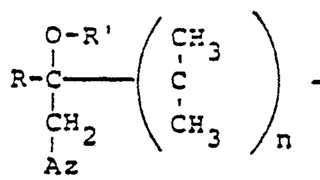

(I)

in welcher

Az für 1,2,4-Triazol-1-yl, 1,2,4-Triazol-4-yl oder Imidazol-1-yl steht,

Het für jeweils gegebenenfalls einfach oder mehrfach, gleich oder verschieden substituiertes Dioxolan-2-yl oder 1,3-Dioxanyl steht, wobei als Substituenten zu nennen sind : Alkyl mit 1 bis 4 Kohlenstoffatomen, sowie jeweils gegebenenfalls einfach oder mehrfach, gleich oder verschieden substituiertes Phenyl und Phenoxyalkyl mit 1 bis 4 Kohlenstoffatomen im Alkylteil, wobei als Phenylsubstituenten zu nennen sind : Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen ;

R für geradkettiges oder verzweigtes Alkyl mit 1 bis 7 Kohlenstoffatomen steht, ferner für jeweils gegebenenfalls einfach oder mehrfach, gleich oder verschieden substituiertes Phenyl, Phenylalkyl mit 1 bis 4 Kohlenstoffatomen im Alkylteil, Phenoxy- und Phenylthioalkyl mit jeweils 1 bis 4 Kohlenstoffatomen im Alkylteil oder für Phenylethenyl steht, wobei als Substituenten an den Phenyl-Gruppen genannt werden : Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und Alkyl mit 1 bis 2 Kohlenstoffatomen substituiertes Phenyl, Phenoxy, Benzyl und Benzyloxy ; weiterhin für jeweils gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen steht, für Cycloalkylmethyl oder -ethyl mit 5 bis 7 Kohlenstoffatomen im Cycloalkylteil und Cyclohexylethenyl steht, schließlich für Alkenyl mit 2 bis 6 Kohlenstoffatomen, für 1,2,4-Triazol-1-yl-methyl 1,2,4-Triazol-4-yl-methyl, Imidazol-1-yl-methyl und Pyrazol-1-yl-methyl steht,

R' für Wasserstoff, für gegebenenfalls durch Phenyl substituiertes Alkyl mit 1 bis 4 Kohlenstoffatomen, wobei der Phenylrest durch die bei R genannten Phenylsubstituenten substituiert sein kann, und für Alkenyl mit 2 bis 4 Kohlenstoffatomen steht ; und

n für die Zahlen 0 oder 1 steht, wobei für den Fall, daß n für 0 steht, Het ein gegebenenfalls wie oben ausgeführt, substituiertes 1,3-Dioxan-5-yl sein muß.

und deren Säureadditionssalze und Metallsalz-Komplexe.

Sie besitzen gute antimikrobiele, insbesondere antimykotische Eigenschaften und sollen daher als Wirkstoffe von antimykotischen Mitteln verwendet werden. Die Verbindungen der Formel (I) besitzen gegebenenfalls ein asymmetrisches Kohlenstoffatom und können deshalb in den beiden optischen Isomerenformen anfallen.

Überraschenderweise zeigen die erfindungsgemäß zu verwendenden heterocyclisch substituierten Hydroxyalkyl-azolyl-Derivate der Formel (I) ein besseres antimykotisches Wirkungsspektrum, insbesondere auch eine bessere, therapeutisch nutzbare *in vivo* Wirksamkeit als die aus dem Stand der Technik bekannten Verbindungen 1-(4-Chlorphenoxy)-3,3-dimethyl-2-(imidazol-1-yl-methyl)-2-butanol, 1-(2-Methylphenoxy)-3,3-dimethyl-2-(imidazol-1-yl-methyl)- bzw. -(1,2,4-triazol-1-yl-methyl)-2-butanol oder 1-(2-Methylphenyl)-4,4-dimethyl-3-(midazol-1-yl-methyl)-3-pentanol, welche konstitutionell und wirkungsmäßig naheliegende Verbindungen sind. Die erfindungsgemäße Verwendung der Stoffe der Formel (I) stellt somit eine Bereicherung der Pharmazie dar.

Die erfindungsgemäßen heterocyclisch substituierten Hydroxyalkyl-azolyl-Derivate sind durch die Formel (I) allgemein definiert. In dieser Formel stehen vorzugsweise

Az für 1,2,4-Triazol-1-yl, 1,2,4-Triazol-4-yl oder Imidazol-1-yl ;

Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten zu nennen sind : Methyl, Ethyl, n-Propyl, Isopropyl sowie gegebenenfalls einfach bis dreifach, gleich oder verschieden durch Fluor, Chlor, Methyl, Trifluormethyl oder Trifluormethoxy substituiertes Phenyl und Phenoxymethyl ;

R für tert.-Butyl, Trimethyl-propyl, Tetramethylpropyl sowie für jeweils gegebenenfalls einfach bis dreifach, gleich oder verschieden substituiertes Phenyl, Benzyl, Phenethyl, Phenoxymethyl, Phenylthiomethyl oder Phenethenyl steht, wobei als Phenylsubstituenten zu nennen sind : Fluor, Chlor, Methyl, Trifluormethyl, Trifluormethoxy, Trifluormethylthio, Hydroxyiminomethyl, 1-Hydroxyiminoethyl, Methoximinomethyl und 1-Methoximinoethyl sowie jeweils gegebenenfalls durch Chlor und/oder Methyl substituiertes Phenyl, Phenoxy, Benzyl und Benzyloxy ; R ferner für jeweils gegebenenfalls einfach bis zweifach, gleich oder verschieden durch Methyl, Ethyl oder Isopropyl substituiertes Cyclohexyl, Cyclohexylmethyl, Cyclohexylethyl oder Cyclohexylethenyl steht ; R weiterhin für Allyl, Dimethylpropenyl, 1,2,4-Triazol-1-yl-methyl, 1,2,4-Triazol-4-yl-methyl, Imidazol-1-yl-methyl oder Pyrazol-1-yl-methyl ; und

R' für Wasserstoff, Methyl, 4-Chlorbenzyl oder Allyl,

wobei für den Fall, daß n für 0 steht, Het ein gegebenenfalls wie oben aufgeführt substituiertes 1,3-Dioxan-5-yl sein muß.

Bevorzugte erfindungsgemäße Verbindungen sind auch Additionsprodukte aus Säure und denjenigen heterocyclisch substituierten Hydroxyalkyl-azolyl-Derivaten der Formel (I), in denen die Substituenten Az, Het, R und R' und der Index n die Bedeutungen haben, die bereits vorzugsweise für diese Substituenten und den Index genannt wurden.

Zur den Säuren, die addiert werden können, gehören vorzugsweise Halogenwasserstoffsäure, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Im einzelnen seien außer den bei den Herstellungsbeispielen genannten Verbindungen die folgenden Verbindungen der neuen Formel (Ia) genannt :

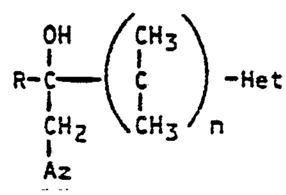

(Ia)

| R | Az | n | Het |
|---|---|---|---|
| 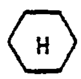 | 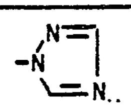 | 1 | 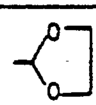 |
| 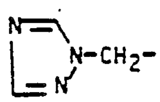 | 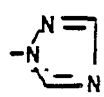 | 1 | 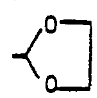 |
| 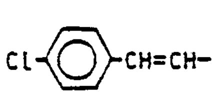 | 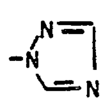 | 1 | 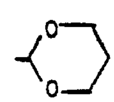 |
| 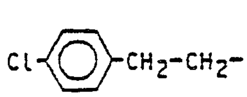 | 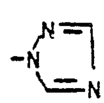 | 1 | 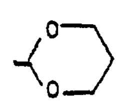 |
| 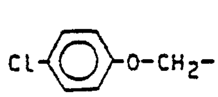 | 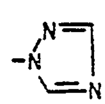 | 1 | 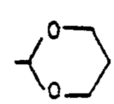 |

(Fortsetzung)

| R | Az | n | Het |
|---|---|---|---|
| 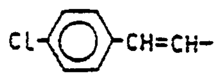 | 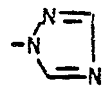 | 1 | 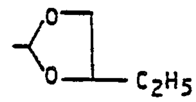 |
| 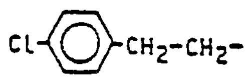 | 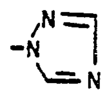 | 1 | 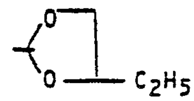 |
| 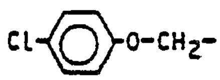 | 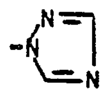 | 1 | 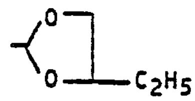 |
| 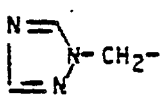 | 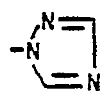 | 1 | 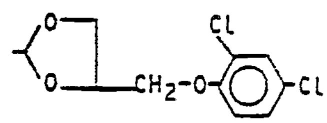 |
| 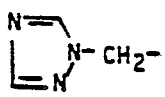 | 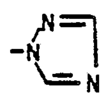 | 1 | 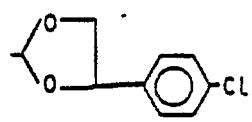 |
| 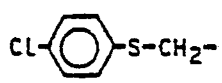 | 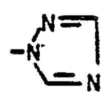 | 1 | 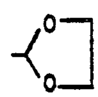 |
| 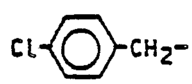 | 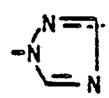 | 1 | 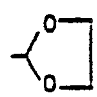 |
| 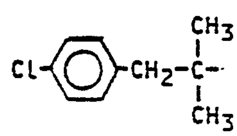 | 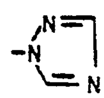 | 1 | 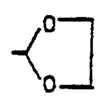 |
| 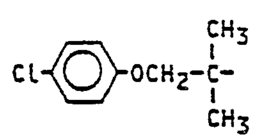 | 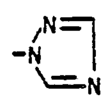 | 1 | 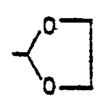 |
| 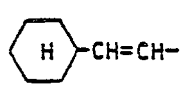 | 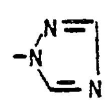 | 1 | 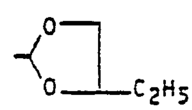 |
| 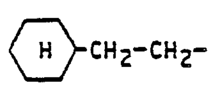 | 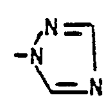 | 1 | 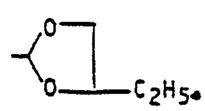 |

(Fortsetzung)

| R | Az | n | Het |
|---|---|---|---|
| 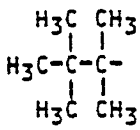 | 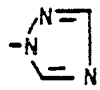 | 1 | 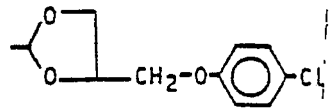 |
| 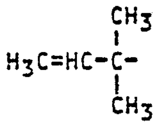 | 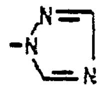 | 1 | 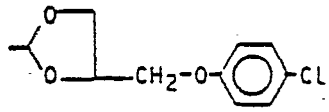 |
| 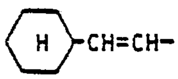 | 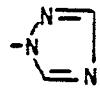 | 0 | 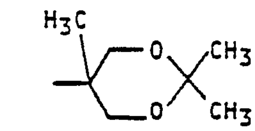 |
| 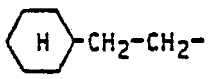 | 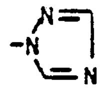 | 0 | 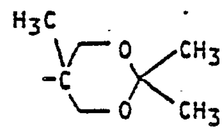 |
| 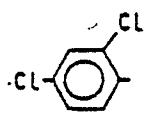 | 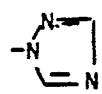 | 1 | 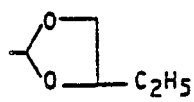 |
| 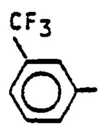 | 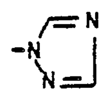 | 1 | 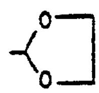 |
| 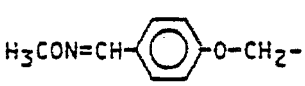 | 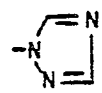 | 1 | 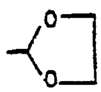 |
| 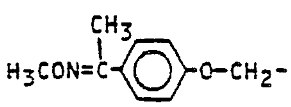 | 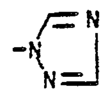 | 1 | 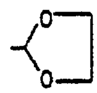 |
| 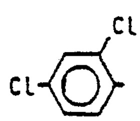 | 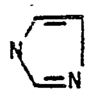 | 0 | 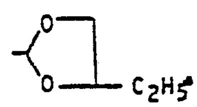 |
| 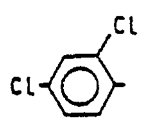 | 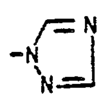 | 0 | 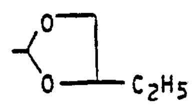 |

Die erfindungsgemäß zu verwendenden, heterocyclisch substituierten Hydroxyalkyl-azolyl-Derivate, ihre Ether- und Ester-Derivate sowie deren Säureadditions-Salze sind noch nicht bekannt. Sie können jedoch gemäß einem eigenen Vorschlag hergestellt werden, indem man

a) Oxirane der Formel (II)

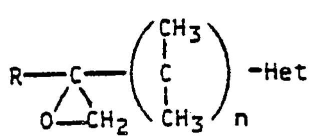

(II)

in welcher Het, R und n die oben angegebene Bedeutung haben,
mit Azolen der Formel (III)

$$M—Az \qquad (III)$$

in welcher
Az die oben angegebene Bedeutung hat und
M für Wasserstoff oder ein Alkylimetallsalz steht,
in Gegenwart eines inerten organischen Lösungsmittels, wie beispielsweise Alkohole, und gegebenenfalls in Gegenwart einer Base, wie beispielsweise Natriumalkoholat oder Kaliumhydroxid bei Temperaturen zwischen 60 °C und 150 °C umsetzt ; oder

b) Azoloketone der Formel (IV)

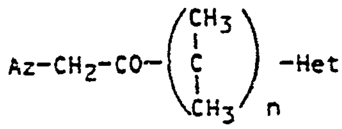

(IV)

in welcher Az, Het und n die oben angegebene Bedeutung haben,
mit einer magnesiumorganischen Verbindung der Formel (V)

$$R—Mg—X \qquad (V)$$

in welcher
R die oben angegebene Bedeutung hat und
X für Chlor, Brom oder Jod steht,
in Gegenwart eines Verdünnungsmittels, wie beispielsweise eines Ethers, bei Temperaturen zwischen 30 °C und 80 °C umsetzt ; oder

c) Azolooxirane der Formel (VI)

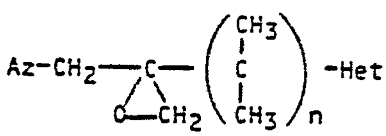

(VI)

in welcher
R die oben angegebene Bedeutung hat und
Me für ein Alkalimetall oder —Mg—X steht, wobei X die oben angegebene Bedeutung hat,
unter den Bedingungen des Verfahrens (a) umsetzt und gegebenenfalls noch

d) die nach Verfahren a), b) oder c) hergestellten Hydroxyverbindungen, in denen R' in Formel (I) für Wasserstoff steht, in Gegenwart eines Verdünnungsmittels in das Alkalimetall-alkoholat überführt und dieses mit einen Halogenid zu den entsprechenden Ether-Derivaten, in denen R' in Formel (I) für gegebenenfalls substituiertes Alkyl oder Phenyl steht, umsetzt.

Außerdem kann an die so erhaltenen Verbindungen der Formel (I) bzw. deren Ester gegebenenfalls anschließend eine Säure addiert werden.

Die Oxirane der Formel (II) sind noch nicht bekannt. Sie können jedoch in allgemein bekannter Art und Weise erhalten werden, indem man Ketone der Formel (VIII)

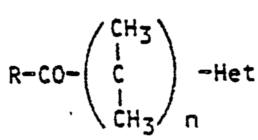

(VIII)

in welcher Het, R und n die oben angegebene Bedeutung haben.
entweder,

α) mit Dimethyloxosulfonium-methylid der Formel (IX)

$$(CH_3)_2 \overset{\delta+}{S}O\overset{\delta-}{C}H_2 \qquad (IX)$$

in an sich bekannter Weise in Gegenwart eines Verdünnungsmittels, wie beispielsweise Dimethylsulfoxid, bei Temperaturen zwischen 20 °C und 80 °C umsetzt (vgl. hierzu die Angaben in J. Am. Chem. Soc. *87*, 1363-1364 (1965)), oder

β) mit Trimethylsulfonium-methylsulfat der Formel (X)

$$\left[(CH_3)_3S^{(+)}\right] CH_3SO_4^{(-)} \qquad (X)$$

in an sich bekannter Weise in Gegenwart eines inerten organischen Lösungsmittels, wie z. B. Acetonitril, und in Gegenwart einer Base, wie z. B. Natriummethylat, bei Temperaturen zwischen 0 °C bis 60 °C, vorzugsweise bei Raumtemperatur; umsetzt (vgl. auch die Angaben in Heterocycles *8*, 397 (1977)).

Die so erhaltenen Oxirane der Formel (III) können gegebenenfalls ohne Isolierung direkt weiter umgesetzt werden.

Die bei der Herstellung der Oxirane der Formel (II) als Ausgangsstoffe benötigten Ketone der Formel (VIII) mit n = 1 sind teilweise bekannt (vgl. z. B. J. Org. Chem. *32*, 404 (1967)) bzw. sind die Gegenstand eigener älterer Patentanmeldungen (vgl. EP-A-97 882 und EP-A-97 881) bzw. können sie in bekannter Art und Weise erhalten werden, indem man 1-(N-Morpholino)-isobuten der Formel (XI)

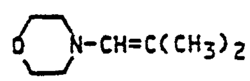 (XI)

mit Chloriden der Formel (XII)

$$R—CO—Cl \qquad (XII)$$

in welcher R die oben angegebene Bedeutung hat,
in Gegenwart eines Lösungsmittels, wie beispielsweise Diethylether, bei Temperaturen zwischen 20 °C und 120 °C umsetzt und die so erhaltenen Keto-Derivate der Formel (XIII)

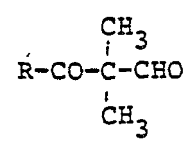 (XIII)

in welcher R die oben angegebene Bedeutung hat,
in üblicher Weise an der Aldehydgruppe mit entsprechenden Diolen in Gegenwart eines inerten organischen Lösungsmittels, wie beispielsweise Toluol, und in Gegenwart einer starken Säuren als Katalysator, wie beispielsweise p-Toluolsulfonsäure, bei Temperaturen zwischen 80 °C und 110 °C derivatisiert.

Die bei der Herstellung der Oxirane der Formel (II) als Ausgangsstoffe benötigten Ketone der Formel (VIII) mit n = 0 sind teilweise bekannt (vgl. z. B. EP-OS 0 043 923) bzw. können sie in bekannter Art und Weise erhalten werden, indem man Aldehydketone (vgl. beispielsweise Houben-Weyl, Methoden der organischen Chemie, Band 7/1, S. 185) der Formel (XIV)

$$R—CO—CHO \qquad (XIV)$$

in welcher R die oben angegebene Bedeutung hat,
oder deren Acetale bzw. deren Ketale der Formel R—C($OCH_3$)$_2$—CHO (zur Herstellung vgl. Bull. Soc. Chim. France, *1971*, S. 2598) in üblicher Weise an der Aldehydgruppe mit entsprechenden Diolen in Gegenwart eines inerten organischen Lösungsmittels, wie beispielsweise Toluol, und in Gegenwart einer starken Säure, wie beispielsweise p-Toluolsulfonsäure, bei Temperaturen zwischen 40 °C und 110 °C umsetzt.

Ketone der Formel (VIII) mit R = gegebenenfalls substituiertes Phenethyl oder Cyclohexylethenyl können auch erhalten werden, indem man entsprechende Benzaldehyde oder Cyclohexylcarbaldehyde mit entsprechenden Methylketonen in üblicher Weise einer Aldolkondensation unterwirft. Die dabei entstehenden Ketone der Formel (VIII) mit R = gegebenenfalls substituiertes Phenethenyl oder Cyclohexylethenyl können gegebenenfalls in üblicher Weise zu Ketonen der Formel (VIII) mit R = gegenenfalls substituiertes Phenethyl oder Cyclohexylethyl hydriert werden (vgl. auch die Herstellungsbeispiele).

Das bei der Verfahrensvariante (α) benötigte Dimethyloxosulfoniummethylid der Formel (IX) ist

ebenfalls bekannt (vgl. J. Amer. Chem. Soc. *87*, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid bzw. Natriumamid in Gegenwart eines Verdünnungsmittels erzeugt.

Das bei der Verfahrensvariante (β) benötigte Trimethylsulfoniummethylsulfat der Formel (X) ist ebenfalls bekannt (vgl. Heterocycles *8*, 397, (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Die Azole der Formel (III) sind allgemein bekannte Verbindungen der organischen Chemie.

Die Azoloketone der Formel (IV) sind teilweise bekannt (vgl. beispielsweise EP-OS 00 43 923), teilweise sind sie jedoch Gegenstand einer eigenen älteren Patentanmeldung (vgl. EP-A-97 881). Die Azoloketone der Formel (IV) können in allgemein bekannter Art und Weise erhalten werden, indem man Halogenmethyl-Ketone der Formel (XV)

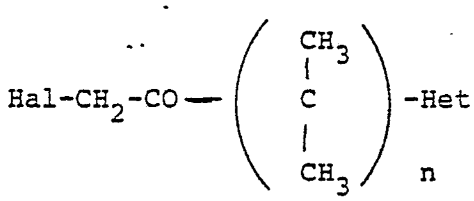

(XV)

in welcher

Het und n die oben angegebene Bedeutung haben und

Hal für Chlor oder Brom steht,

in üblicher Weise mit 1,2,4-Triazol oder Imidazol in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 °C und 150 °C umsetzt.

Die bei der Herstellung der Azoloketone der Formel (IV) als Ausgangsstoffe benötigten Halogenmethyl-Ketone der Formel (XV) mit n = 1 sind noch nicht bekannt ; auch sie sind Gegenstand der eigenen älteren Patentanmeldung (vgl. EP-A-97 881). Sie können gemäß der oben beschriebenen Herstellung der Ketone der Formel (VIII) mit n = 1 erhalten werden.

Die bei der Herstellung der Azoloketone der Formel (IV) als Ausgangsstoffe benötigten Halogenmethyl-Ketone der Formel (XV) mit n = 0 sind teilweise bekannt (vgl. die EP-OS 0 043 923) ; sie können beispielsweise in bekannter Art und Weise durch Halogenierung, wie beispielsweise mit N-Bromsuccinimid, entsprechender Ketone der Formel (VIII) erhalten werden.

Die magnesium-organischen Verbindungen der Formel (V) sind allgemein bekannte Verbindungen der organischen Chemie ; bzw. werden sie in allgemein bekannter Art und Weise erhalten.

Die Azolooxirane der Formel (VI) sind noch nicht bekannt ; sie können jedoch in allgemein bekannter Art und Weise erhalten werden, indem man Azoloketone der Formel (IV) entsprechend den oben angegebenen Verfahrensvarianten (α) und (β) epoxidiert.

Die Phenole und Thiophenole sowie die metallhaltigen Verbindungen der Formel (VII) sind allgemein bekannte Verbindungen der organischen Chemie.

Zur Herstellung der Ether der Verbindungen der Formel (I) wird zweckmäßigerweise so verfahren, daß man Verbindungen der Formel (I) in einem geeigneten inerten organischen Lösungsmittel mittels Alkalimetall-hydrid oder -amid in das Alkalimetall-alkoholat überführt und dieses ohne Isolierung sofort mit einem entsprechenden Halogenid, wie insbesondere einem Alkylhalogenid, umsetzt, wobei unter Austritt von Alkalihalogenid die Ether der Verbindungen der Formel (I) in einem Arbeitsgang erhalten werden.

In einer bevorzugten Ausführungsform werden zweckmäßigerweise die Herstellung der Alkoholate sowie deren weitere Umsetzung mit einem Halogenid in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphonium-Verbindungen, durchgeführt, wobei in der organischen Phase oder an der Grenzfläche die Alkoholate entstehen und mit den in der organischen Phase befindlichen Halogeniden umgesetzt werden.

Die nach den erfindungsgemäßen Verfahren (a), (b) und (c) erhältlichen Hydroxiverbindungen, in denen R' in Formel (I) für Wasserstoff steht, können auch in die entsprechenden Ester überführt werden.

Zur Herstellung der Ester wird zweckmäßigerweise so verfahren, daß man Verbindungen der Formel (I) z. B. mit Säurehalogeniden in Gegenwart eines inerten organischen Lösungsmittels, wie beispielsweise Essigester, bei Temperaturen zwischen 0 °C und 100 °C umsetzt ; oder mit Säureanhydriden in Gegenwart eines inerten organischen Lösungsmittels, wie beispielsweise Methylenchlorid oder Überschuß an Säureanhydrid, und in Gegenwart eines Katalysators, wie beispielsweise Natriumacetat, bei Temperaturen zwischen 0 °C und 150 °C umsetzt.

Die Verbindungen der Formel (I) sowie ihre Ester können auch in Säureadditions-Salze überführt werden.

Die Säureadditions-Salze der Verbindungen der Formel (I) können in einfacher Weise nach üblichen

Salzbindungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I), deren Ester-Derivate und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bi-phasische Pilze, z. B. gegen Candida-Arten, wie Candida albicans, Epidermophyten-Arten, wie Epidermophyton floccosum. Aspergillus-Arten, wie Aspergillus niger und Aspergillus fumigatus, Trichophyton-Arten, wie Trichophyton mentagrophytes, Microsporon-Arten, wie Microsporon felineum sowie Torulospsis-Arten, wie Torulospsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden :

Dermatomykosen und Systemmykosen durch Trichophyton mentagrophytes und andere Trichophytonarten, Microsporonarten, Epiderophyton floccosum, Sproßpilze und biphasische Pilze sowie Schimmelpilze hervorgerufen.

Als Indikationsgebiete in der Tiermedizin können beispielsweise aufgeführt werden :

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigenten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tablette, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Plyethylenglykole und Fettsäureester des

Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffen wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemößе Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesonder intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderliche sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittel sowie dem Zeitraum bzw. Intervall, innerhalb welchem die Verabreichung erfolgt. So kann es in einigen Fällen ausreichend sein, mit weniger als der obengenannten Menge Wirkstoffe auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

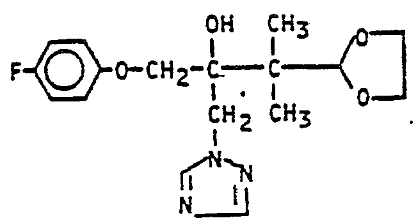

(Verfahren a)

Eine Lösung von 17,6 g (0,0623 Mol) 2-[2-(1,3-Dioxolan-2-yl)-prop-2-yl]-2-(4-fluorphenoxymethyl)-oxiran in 30 ml n-Propanol wird bei Raumtemperatur zu einer Lösung von 4,93 g (0,0715 Mol) 1,2,4-Triazol und 0,36 g (0,0065 Mol) Kaliumhydroxid in 30 ml n-Propanol getropft. Das Reaktionsgemisch wird 2 Tage unter Rückfluß erhitzt und anschließend eingeengt. Der Rückstand wird in Essigester aufgenommen, zweimal mit Wasser und einmal mit gesättigter Kochsalzlösung gewaschen, über Natriumsulfat getrocknet und eingeengt. Der Rückstand wird säulenchromatographisch gereinigt (Dichlormethan/Essigester = 3 : 1) und aus wenig Ether umkristallisiert.

Man erhält 10,2 g (47 % der Theorie) 3-(1,3-Dioxolan-2-yl)-1-(4-fluorphenoxy)-3-methyl-2-(1,2,4-triazol-1-yl-methyl)-2-butanol vom Schmelzpunkt 102 °C bis 104 °C.

Herstellung des Ausgangsproduktes

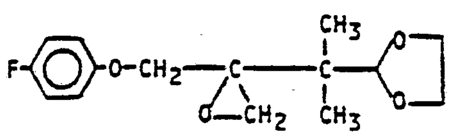

Eine Suspension von 16,47 g (0,0748 Mol) Trimethylsulfoxoniumiodid in 20 ml absolutem Dimethylsulfoxid wird bei Raumtempratur mit 8,4 g (0,0748 Mol) Kalium-tert.-butylat versetzt. Man läßt 30 Minuten nachrühren und tropft dann eine Lösung von 3-(1,3-Dioxolan-2-yl)-1-(4-fluorphenoxy)-3-methyl-2-butanon in 20 ml absolutem Toluol zu. Das Reaktionsgemisch wird über Nacht bei Raumtemperatur gerührt, danach 2 Stunden auf 50 °C erhitzt, abgekühlt und mit Wasser und Toluol versertzt. Die Toluolphase wird abgetrennt, zweimal mit Wasser und einmal mit gesättigter Natriumchloridlösung gewaschen, über Natriumsulfat getrocknet und eingeengt.

Man erhält 17,6 g (91 % der Theorie 2-[2-(1,3-Dioxolan-2-yl)-prop-2-yl]-2-(4-fluorphenoxymethyl)-oxiran als Öl, das direkt weiter umgesetzt wird.

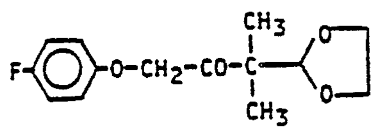

Ein Gemisch aus 16,7 g (0,15 Mol) 4-Fluorphenol, 29 g (0,15 Mol) 1-Chlor-3-(1,3-dioxolan-2-yl)-3-methyl-2-butanon und 23,4 g (0,17 Mol) gepulvertem Kaliumcarbonat in 300 ml Methylethylketon wird 16 Stunden unter Rückfluß erhitzt. Man läßt abkühlen und filtriert. Das Filtrat wird eingeengt, der Rückstand mit Dichlormethan aufgenommen, einmal mit 5 %iger Natronlauge und einmal mit Wasser gewaschen, über Natriumsulfat getrocknet, eingeengt und destilliert. Man erhält 29 g (72 % der Theorie) 3-(1,3-Dioxolan-2-yl)-1-(4-fluorphenoxy)-3-methyl-2-butanon vom Siedepunkt 143 °C bis 145 °C/0,1 mbar.

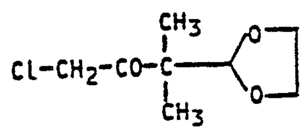

204 g (1,38 Mol) 4-Chlor-2-dimethyl-3-keto-butanal werden mit 93 g (1,5 Mol) Ethylenglykol und 0,7 g p-Toluolsulfonsäure in 400 ml Methylenchlorid während 3 Stunden am Wasserabscheider erhitzt. Die organische Phase wird mit 150 ml 5 %iger Natronlauge und danach mit 400 ml Wasser extrahiert. Das Lösungsmittel wird abdestilliert und der Rückstand im Wasserstrahlvakuum destilliert.

Man erhält 211 g (79,8 % der Theorie) 1-Chlor-3-(dioxolan-2-yl)-3-methyl-2-butanon vom Siedepunkt 127 °C bis 128 °C/14 mbar.

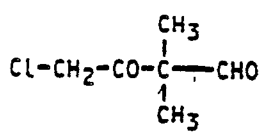

210 g (1,5 Mol) 1-(N-Morpholino)-isobuten werden innerhalb einer Stunde zu 169 g (1,5 Mol) Chloracetylchlorid, gelöst in 350 ml Diethylether, bei 5 °C zugetropft. Nach beendeter Zugabe rührt man weitere 3 Stunden unter Rückflußkühlung. Die Lösung wird auf 100 g Eis gegossen, mit wäßriger Natriumhydrogencarbonatlösung auf pH 5 gebracht und die Etherphase abgetrennt. Die Wasserphase wird mit 100 ml Diethylether extrahiert, die organischen Phasen werden vereinigt, über Natriumsulfat getrocknet, das Lösungsmittel abdestilliert und der Rückstand im Wasserstrahlvakuum destilliert.

Man erhält 136,4 g (61 % der Theorie) 4-Chlor-2,2-dimethyl-3-ketobutanal vom Siedepunkt 95 °C bis 98 °C/14 mbar.

Beispiele 2 und 3

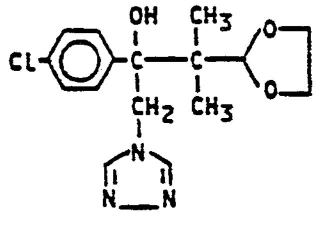

(Beispiel 2)

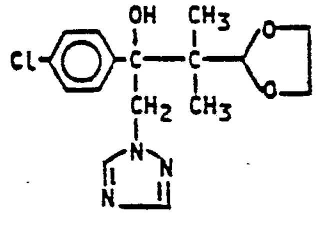

(Beispiel 3)

(Verfahren a)

Eine Lösung von 26,8 g (0,1 Mol) 2-(4-Chlorphenyl)-2-[2-(1,3-dioxolan-2-yl)-prop-2-yl]-oxiran, 7,6 g (0,11 Mol) 1,2,4-Triazol und 0,5 g Kaliumhydroxid in 200 ml absolutem Butanol wird 18 Stunden unter Rückfluß erhitzt. Man läßt auf Raumtemperatur abkühlen und versetzt mit 800 ml Wasser. Die organische Phase wird abgetrennt, über Natriumsulfat getrocknet und eingeengt. Der Rückstand wird mit 200 ml Isopropylether/Essigester (10 : 1) verrührt.

Der ausfallende Feststoff wird abfiltriert und getrocknet. Man erhält 4,5 g (13,3 % der Theorie) 3-(1,3-Dioxolan-2-yl)-2-(4-chlorphenyl)-3-methyl-1-(1,2,4-triazol-4-yl)-2-butanol (Beispiel 2) vom Schmelzpunkt 180 °C bis 182 °C.

Das Filtrat wird eingeengt und der Rückstand zunächst mit 350 ml Aceton aufgenommen und dann mit 8 g 1,5-Naphthalindisulfonsäure in 30 ml Aceton versetzt. Man läßt 6 Stunden bei 0 °C rühren, saugt den Feststoff ab und versetzt mit gesättigter, wäßriger Natriumhydrogencarbonat/Methylen-Lösung. Die organische Phase wird abgetrennt, über Natriumsulfat getrocknet und eingeengt.

Man erhält 19 g (56,2 % der Theorie) 3-(1,3-Dioxolan-2-yl)-2-(4-chlorphenyl)-3-methyl-1-(1,2,4-triazol-1-yl)-2-butanol (Beispiel 3) als Öl.

Herstellung des Ausgangsproduktes

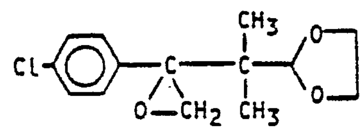

29,5 g (0,208 Mol) Methyljodid werden zu 13,7 g (0,22 Mol) Dimethylsulfid in 130 ml absolutem Dimethylsulfoxid und 120 ml absolutem Tetrahydrofuran getropft, wobei die Temperatur auf ca. 30 °C steigt. Man läßt 5 Stunden nachrühren und versetzt dann mit einer Lösung von 33 g (0,13 Mol) 4-Chlorphenyl-2-[2-(1,3-dioxolan-2-yl)-prop-2-yl]-keton in 100 ml absolutem Toluol. Innerhalb einer Stunde werden 9,5 g Natriummethylat portionsweise zugegeben. Man läßt das Reaktionsgemisch 3 Stunden nachrühren und gibt innerhalb von 30 Minuten nochmals 5,6 g Natriummethylat in 2 Portionen zu. Man läßt über Nacht rühren und gibt das Reaktionsgemisch auf 700 ml Eiswasser. Die organische Phase wird abgetrennt und die wäßrige Phase mit 200 ml Toluol ausgeschüttelt. Die vereinigten organischen Phasen werden zweimal mit je 1 000 ml Wasser gewaschen, über Natriumsulfat getrocknet und eingeengt. Der ölige Rückstand wird im Vakuum entgast.

Man erhält 26,8 g 2-(4-Chlorphenyl)-2-[2-(1,3-dioxolan-2-yl)-prop-2-yl]-oxiran als Öl, das direkt weiter umgesetzt wird.

## Beispiel 4

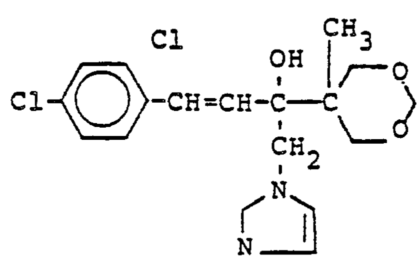

(Verfahren a)

13,6 g (0,2 Mol) Imidazol werden portionsweise zu 6 g (80 %ig, 0,2 Mol) Natriumhydrid in 200 ml absolutem Dimethylformamid gegeben, wobei die Temperatur auf ca. 45 °C ansteigt. Man läßt 30 Minuten nachrühren und gibt dann 34 g (0,108 Mol) 2-(2,4-Dichlorphenethenyl)-2-(5-methyl-1,3-dioxan-5-yl)-oxiran in 50 ml absolutem Dimethylformamid zu. Das Reaktionsgemisch wird 4 Stunden bei 80 °C gerührt. Man läßt abkühlen und gibt auf 800 ml Eiswasser/600 ml Methylenchlorid. Man läßt 45 Minuten nachrühren, trennt die Methylenchloridphase ab, wäscht zweimal mit Wasser, trocknet über Natriumsulfat und engt ein. Der Rückstand wird aus Ether umkristallisiert.

Man erhält 10,5 g (25,4 % der Theorie) 1-(2,4-Dichlorphenyl)-4-(imidazol-1-yl)-3-(5-methyl-1,3-dioxan-5-yl)-1-buten-3-ol vom Schmelzpunkt 142 °C bis 144 °C.

Herstellung des Ausgangsproduktes

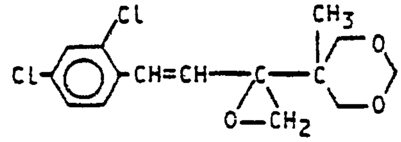

56,8 g (0,4 Mol) Methyljodid werden zu 26,4 g (0,425 Mol) Dimethylsulfid in 180 ml absolutem Dimethylsulfoxid und 175 ml absolutem Tetrahydrofuran getropft, wobei die Temperatur auf ca. 35 °C steigt. Man läßt 16 Stunden nachrühren und versetzt dann mit einer Lösung von 75,2 g (0,25 Mol) 2,4-Dichlorphenethenyl-(5-methyl-1,3-dioxan-5-yl)-keton in 200 ml absolutem Toluol. Bei 0 °C werden 17,4 g (0,3 Mol) Natriummethylat portionsweise eingetragen. Man läßt 3 Stunden nachrühren, versetzt nochmals mit 10,8 g (0,2 Mol) Natriummethylat und läßt über Nacht rühren. Das Reaktionsgemisch wird mit 250 ml Wasser versetzt, die Toluolphase abgetrennt und die wäßrige Phase zweimal mit je 150 ml Toluol extrahiert. Die vereinigten Toluolphasen werden dreimal mit je 700 ml Wasser gewaschen, über Natriumsulfat getrocknet und eingeengt. Der ölige Rückstand wird im Vakuum entgast.

Man erhält 65 g 2-(2,4-Dichlorphenethenyl)-2-(5-methyl-1,3-dioxan-5-yl)-oxiran als Öl, das direkt weiter umgesetzt wird.

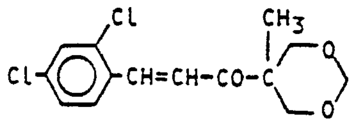

70 g (0,4 Mol) 2,4-Dichlorbenzaldehyd und 57,5 g (85 %ig, 0,4 Mol) Methyl-(5-methyl-1,3-dioxan-5-yl)-keton in 140 ml Ethanol und 50 ml Wasser werden tropfenweise mit 35 ml 10 %iger Natronlauge versetzt. Man läßt die Reaktionsmischung 8 Stunden bei Raumtemperatur nachrühren und gibt anschließend auf 400 ml Methylenchlorid. Die organische Phase wird abgetrennt, mit Wasser gewaschen, über Natriumsulfat getrocknet und eingeengt. Der ölige Rückstand kristallisiert nah Verreiben mit Ether.

Man erhält 80 g 2,4-Dichlorphenethenyl-(5-methyl-1,3-dioxan-5-yl)-keton vom Schmelzpunkt 100 °C bis 102 °C.

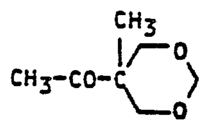

360 g (5 Mol) Methylethylketon und 225 g (2,5 Mol) Trioxan werden in 1 000 ml Chloroform unter Zugabe von 40 ml konzentrierter Schwefelsäure 2 Stunden unter Rückfluß erhitzt. Man läßt abkühlen, versetzt mit 2 l Wasser und läßt 10 Minuten nachrühren. Die organische Phase wird abgetrennt, in gesättigte, wäßrige Natriumhydrogencarbonatlösung eingerührt und das Gemisch erneut während 10 Min. nachgerührt. Die organische Phase wird abgetrennt, über Natriumsulfat getrocknet und eingeengt. Der Rückstand wird im Vakuum destilliert.

Man erhält 199 g Methyl-(5-methyl-1,3-dioxan-5-yl)-keton vom Siedepunkt 50 °C bis 52 °C/0,8 mbar.

Beispiel 5

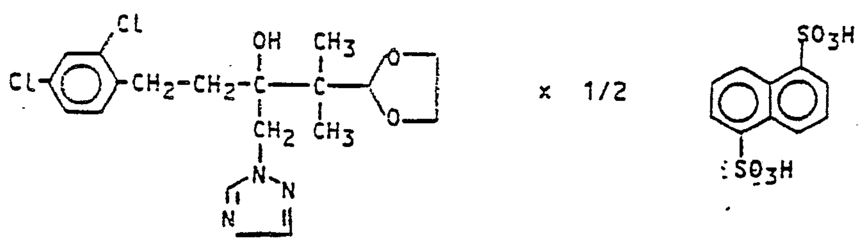

(Verfahren a/Salzbildung)

13,8 g (0,2 Mol) 1,2,4-Triazol werden portionsweise zu einer Suspension von 6 g (80 %ig, 0,2 Mol) Natriumhydrid in 330 ml absolutem Dimethylformamid gegeben. Man läßt 30 Minuten nachrühren und versetzt dann mit 2-(2,4-Dichlorphenylethyl)-2-[2-(1,3-dioxolan-2-yl)-prop-2-yl]-oxiran in 80 ml Dimethylformamid. Das Reaktionsgemisch wird 4 Stunden bei 80 °C gerührt. Danach läßt man abkühlen und gibt auf 600 ml Eiswasser/800 ml Methylenchlorid. Die Methylenchloridphase wird abgetrennt, zweimal mit je 1 500 ml Wasser gewaschen, über Natriumsulfat getrocknet und eingeengt. Der ölige Rückstand wird in 400 ml Aceton aufgenommen und bei 0 °C mit 14,4 g 1,5-Naphthalindisulfonsäure in 40 ml Aceton versetzt. Man läßt 5 Stunden nachrühren und saugt den entstandenen Niederschlag ab.

Man erhält 43,1 g (29,1 % der Theorie) 1-(2,4-Dichlorphenyl)-4-(1,3-dioxolan-2-yl)-4-methyl-3-(1,2,4-triazol-1-yl-methyl)-3-pentanol-naphthalindisulfonat-(1,5) vom Schmelzpunkt 183 °C.

Herstellung des Ausgangsproduktes

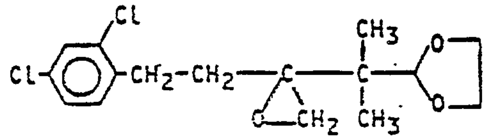

Gemäß Beispiel 3 erhält man durch Umsetzung von 83 g (0,262 Mol) 2,4-Dichlorphenylethyl-2-(1,3-dioxolan-2-yl)-prop-2-yl-keton mit 28,2 g (0,455 Mol) Dimethylsulfid/60 g Methyljodid, 90 g 2-(2,4-Dichlorphenylethyl)-2-[2-(1,3-dioxolan-2-yl)-prop-2-yl]-oxiran als Öl, das direkt weiter umgesetzt wird.

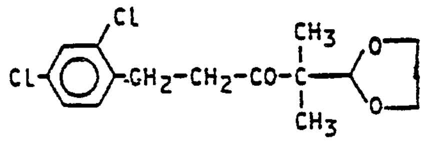

100 g 2,4-Dichlorphenylethenyl-2-(1,3-dioxolan-2-yl)-prop-2-yl-keton werden mit 10 g Raney-Nickel in 600 ml Tetrahydrofuran 25 Minuten bei einem Druck von 55 bar auf 25 °C erhitzt. Das Reaktionsgemisch wird anschließend eingeengt und der Rückstand im Vakuum destilliert.

Man erhält 83 g 2,4-Dichlorphenylethyl-2-(1,3-dioxolan-2-yl)-prop-2-yl-keton vom Siedepunkt 148 °C/0,1 mbar.

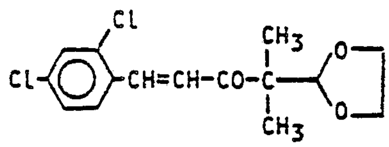

196 g (1,12 Mol) 2,4-Dichlorbenzaldehyd und 178 g (0,125 Mol) Methyl-2-(1,3-dioxolan-2-yl)-prop-2-yl-keton in 400 ml Ethanol und 140 ml Wasser werden tropfenweise mit 95 ml 10 %iger Natronlauge versetzt. Man läßt das Reaktionsgemisch 10 Stunden nachrühren, saugt den entstandenen Feststoff ab und wäscht ihn mit Ethanol.

Man erhält 309 g 2,4-Dichlorphenylethenyl-2-(1,3-dioxolan-2-yl)-prop-2-yl-keton vom Schmelzpunkt 92 °C bis 93 °C.

## Beispiel 6

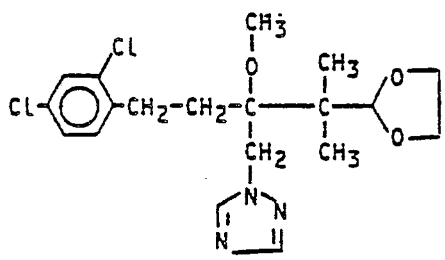

(Verfahren d/Etherbildung)

Zu 18 g (0,045 Mol) 1-(2,4-Dichlorphenyl)-4-(1,3-dioxan-2-yl)-4-methyl-3-(1,2,4-triazol-1-yl-methyl)-3-pentanol in 150 ml absolutem Dioxan werden 1,4 g (0,046 Mol) Natriumhydrid zugegeben. Man läßt 5 Stunden bei Raumtemperatur nachrühren und versetzt mit 7,1 g (0,05 Mol) Methyljodid. Danach wird über Nacht nachgerührt und erneut mit 0,7 g (0,023 Mol) Natriumhydrid und 3,5 g (0,025 Mol) Methyljodid versetzt. Nach nochmaligem Nachrühren wird eingeengt, der ölige Rückstand in Methylenchlorid aufgenommen, zweimal mit je 600 ml Wasser nachgewaschen, über Natriumsulfat getrocknet und eingeengt. Der Rückstand wird aus Isopropylether umkristallisiert.

Man erhält 12 g (64,4 % der Theorie) 1-(2,4-Dichlorphenyl)-4-(1,3-dioxan-2-yl)-3-methoxy-4-methyl-3-(1,2,4-triazol-1-yl-methyl)-pentan vom Schmelzpunkt 130 °C bis 132 °C.

In analoger Weise und entsprechend den erfindungsgemäßen Verfahrensangaben werden die nachfolgenden Verbindungen der allgemeinen Formel (Ia) erhalten :

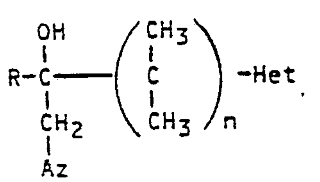

(Ia)

| Beisp. Nr. | R | Az | n | Het | Schmelzpunkt (°C) bzw. $n_D^{20}$ |
|---|---|---|---|---|---|
| 7 | 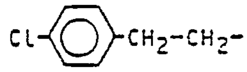 | 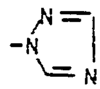 | 1 | 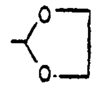 | 179 (x 1/2 NDS) |
| 8 | 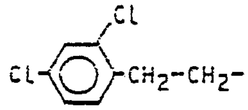 | 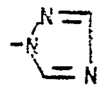 | 0 | 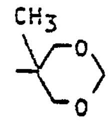 | 119 - 121 |
| 9 | 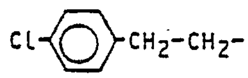 | 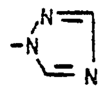 | 0 | 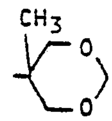 | 125 - 127 |
| 10 | 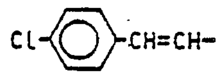 | 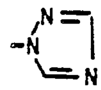 | 1 | 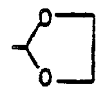 | 116 - 118 |
| 11 | 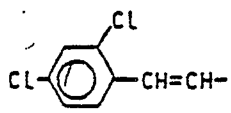 | 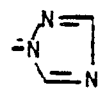 | 1 | 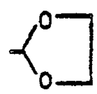 | 93 |
| 12 | 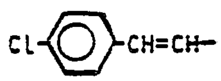 | 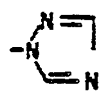 | 0 | 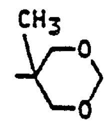 | 182 - 184 |
| 13 | 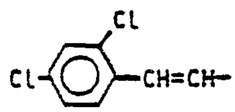 | 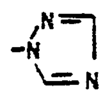 | 0 | 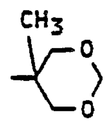 | 171 - 173 |
| 14 | 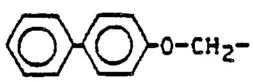 | 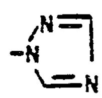 | 1 | 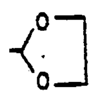 | 106 - 107 |
| 15 | 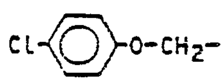 | 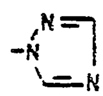 | 1 | 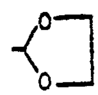 | 105 - 107 |
| 16 | 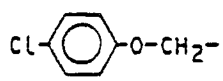 | 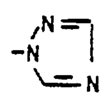 | 0 | 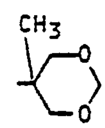 | 102 - 107 |
| 17 | 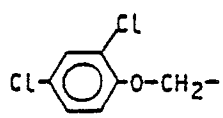 | 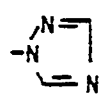 | 0 | 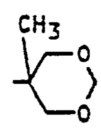 | 117 - 122 |
| 18 | 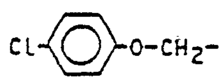 | 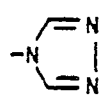 | 0 | 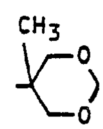 | 220 - 225 |
| 19 | 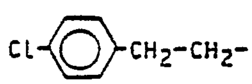 | 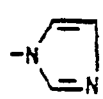 | 1 | 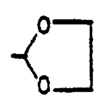 | 93 |

(Fortsetzung)

| Beisp. Nr. | R | Az | n | Het | Schmelzpunkt (°C) bzw. $n_D^{20}$ |
|---|---|---|---|---|---|
| 20 | 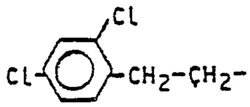 | 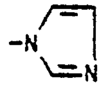 | 1 | 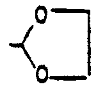 | 140-143 (x 1/2 NDS) |
| 21 | 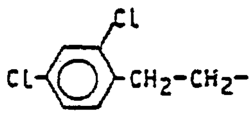 | 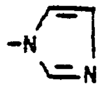 | 0 | 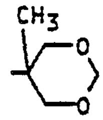 | 122 - 124 |
| 22 | 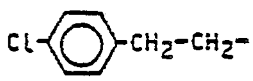 | 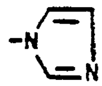 | 0 | 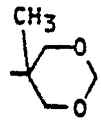 | 68 - 73 |
| 23 | 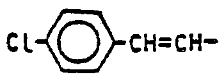 | 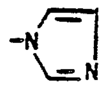 | 1 | 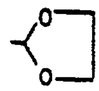 | 164 - 166 |
| 24 | 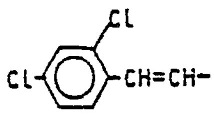 | 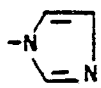 | 1 | 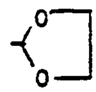 | 122 - 125 |
| 25 | 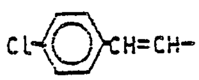 | 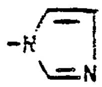 | 0 | 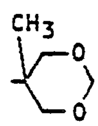 | 192 - 195 |
| 26 | 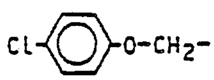 | 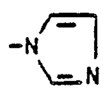 | 0 | 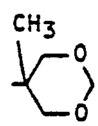 | 141 - 144 |
| 27 | 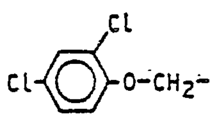 | 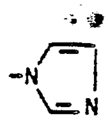 | 0 | 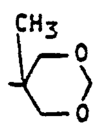 | 176 - 177 |
| 28 | 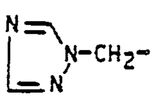 | 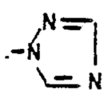 | 1 | 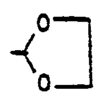 | 1,5240 |
| 29 | 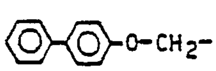 | 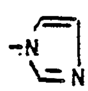 | 1 | 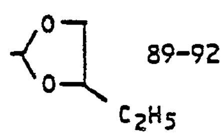 | 89-92 |
| 30 | 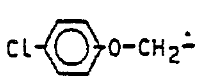 | 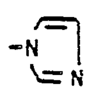 | 1 | 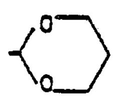 | 129-30 |
| 31 | 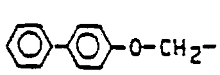 | 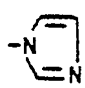 | 1 | 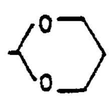 | 138-39 |

NDS = 1,5-Naphthalindisulfonsäure

(Fortsetzung)

| Beisp. Nr. | R | Az | n | Het | Schmp. (°C) bzw. $n_D^{20}$ |
|---|---|---|---|---|---|
| 32 | 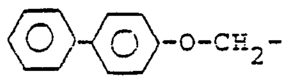 | 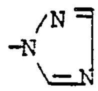 | 1 | 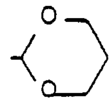 | 149 |
| 33 | 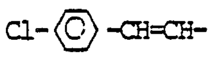 | 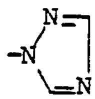 | 1 | 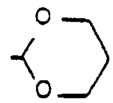 | 147–49 |
| 34 | 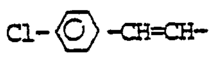 | 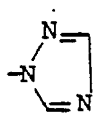 | 1 | 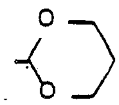 | 152–55 |
| 35 | 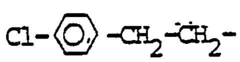 | 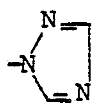 | 1 | 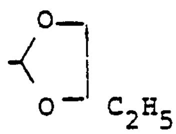 | zähfl. Öl |
| 36 | 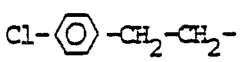 | 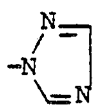 | 1 | 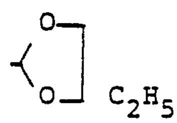 | " " |
| 37 | 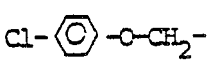 | 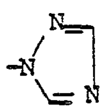 | 1 | 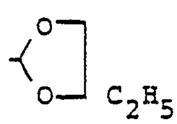 | 169–72 |
| 38 | 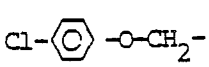 | 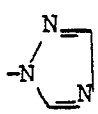 | 1 | 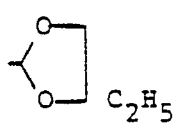 | zähfl. Öl |
| 39 | 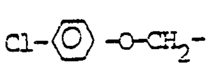 | 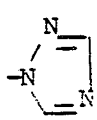 | 1 | 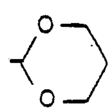 | zähfl. Öl |
| 40 | 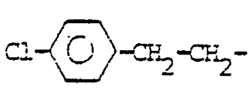 | 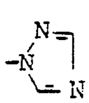 | 1 | 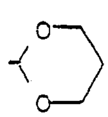 | 110–12 |

(Fortsetzung)

| Beisp. Nr. | R | Az | n | Het | Schmp. (°C) bzw. $n_D^{20}$ |
|---|---|---|---|---|---|
| 41 | 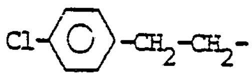 | 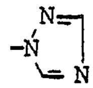 | 1 | 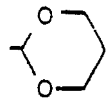 | 117-19 |
| 42 | 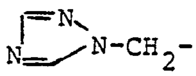 | 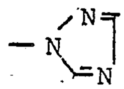 | 1 | 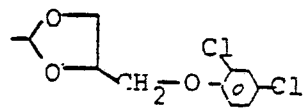 | 1,5461 |
| 43 | 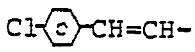 | " | 1 | 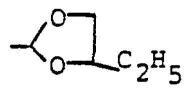 | 80 |
| 44 | " | 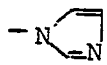 | 1 | " | 88-90 |
| 45 | 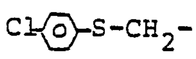 | 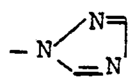 | 1 | 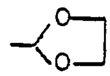 | zähes Oel |
| 46 | 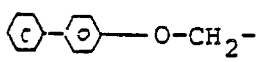 | " | 1 | 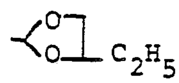 | 105 |
| 47 | 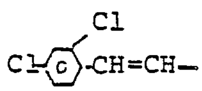 | 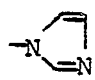 | 0 | 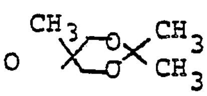 | zähes Oel |
| 48 | " | 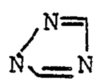 | 0 | " | zähes Oel |
| 49 | 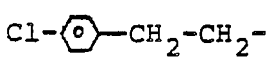 | 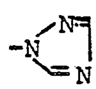 | 1 | 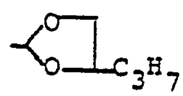 | zähes Oel |
| 50 | 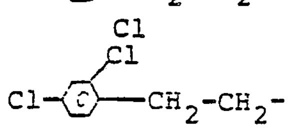 | 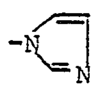 | 1 | " | 115 |
| 51 | " | 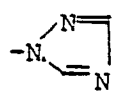 | 1 | " | zähes Oel |
| 52 | 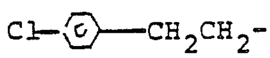 | 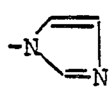 | 1 | 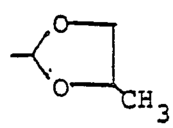 | zähes Oel |
| 53 | 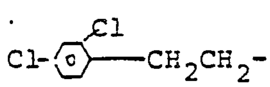 | " | 1 | " | zähes Oel |
| 54 | 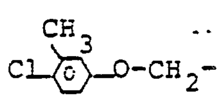 | " | 1 | " | zähes Oel |

(Fortsetzung)

| Beisp. Nr. | R | Az | n | Het | Schmp. (°C) bzw. $n_D^{20}$ |
|---|---|---|---|---|---|
| 55 | " | " | 1 | 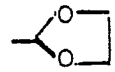 | 123 |
| 56 | " | 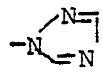 | 1 | " | 81 |
| 57 | 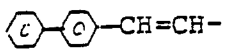 | 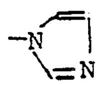 | 1 | 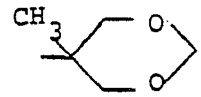 | 146 |
| 58 | " | 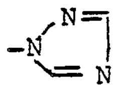 | 0 | " | 170 |
| 59 | 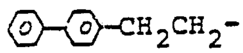 | " | 0 | " | 98 |
| 60 | " | 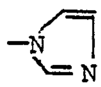 | 0 | " | 131 |
| 61 | 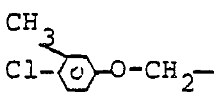 | 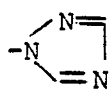 | 1 | 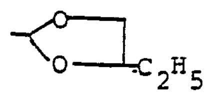 | zähes Oel |
| 62 | " | 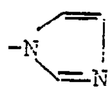 | 1 | " | zähes Oel |
| 63 | 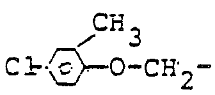 | 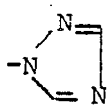 | 1 | " | zähes Oel |
| 64 | 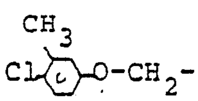 | " | 1 | 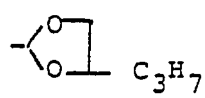 | zähes Oel |
| 65 | 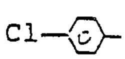 | 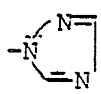 | 1 | 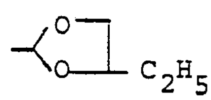 | zähes Oel |
| 66 | 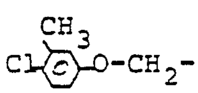 | " | 1 | 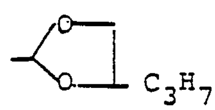 | zähes Oel |
| 67 | 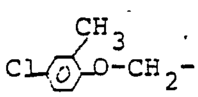 | " | 1 | " | zähes Oel |
| 68 | " | 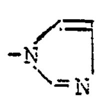 | 1 | " | zähes Oel |

(Fortsetzung)

| Beisp. Nr. | R | Az | n | Het | Schmp. (°C) bzw. $n_D^{20}$ |
|---|---|---|---|---|---|
| 69 | 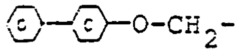 | 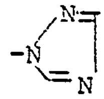 | 0 | " | 113-115 |
| 70 | 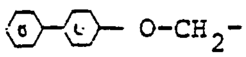 | 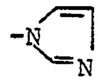 | 0 | " | 169-171 |
| 71 | 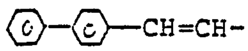 | 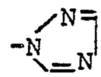 | 1 | 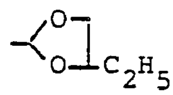 | zähes Oel |
| 72 | " | 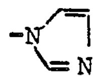 | 1 | " | 132-135 |
| 73 | 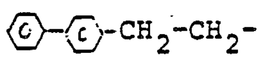 | 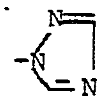 | 1 | " | zähes Oel |
| 74 | " | 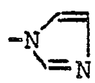 | 1 | " | zähes Oel |
| 75 | 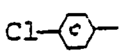 | 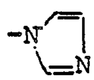 | 1 | " | zähes Oel |
| 76 | 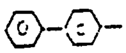 | 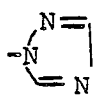 | 1 | " | zähes Oel |
| 77 | 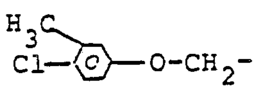 | 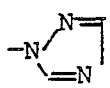 | 1 | 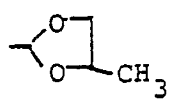 | zähes Oel |
| 78 | 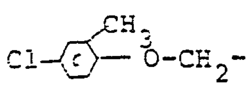 | 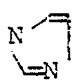 | 1 | 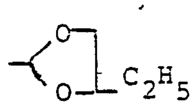 | zähes Oel |
| 79 | 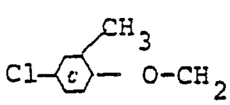 | 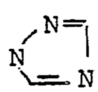 | 1 | 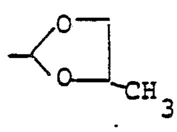 | zähes Oel |
| 80 | 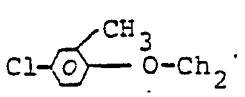 | 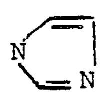 | 1 | 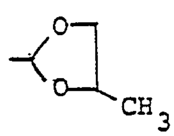 | zähes Oel |
| 81 | 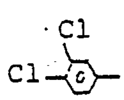 | 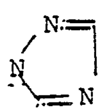 | 1 | 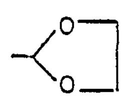 | zähes Oel |

(Fortsetzung)

| Beisp. Nr. | R | Az | n | Het | Schmp. (°C) bzw. $n_D^{20}$ |
|---|---|---|---|---|---|
| 82 | 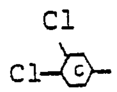 | 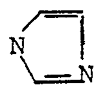 | 1 | 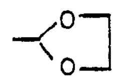 | 138 |
| 83 | 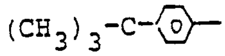 | 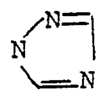 | 1 | 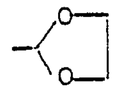 | zähes Oel |
| 84 | 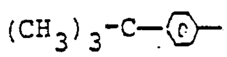 | 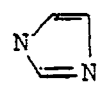 | 1 | 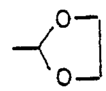 | 164-166 |

## Verwendungsbeispiele

In den nachfolgenden Beispielen werden die nachstehend angegebenen Verbindungen als Vergleichssubstanzen eingesetzt :

(A) 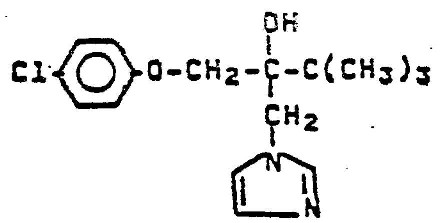

(B) 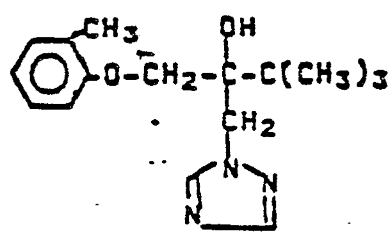

(C) 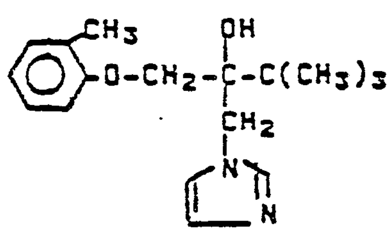

(D) 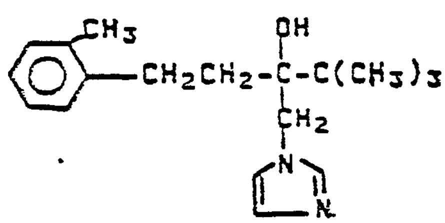

## Beispiel A

Antimykotische in-vitro-Wirksamkeit

Versuchsbeschreibung :

Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze : Sabourand's milieu d'épreuve
b) für Hefen : Fleischextrakt-Traubenzucker-Bouillon.

Die Bebrütungstemperatur betrug 20 °C, die Betrütungsdauer lag bei 24 bis 96 Stunden bei Hefen und 96 Stunden bei Dermatophyten und Schimmelpilzen.

In diesem Test zeigten z. B. die erfindungsgemäßen Verbindungen 3, 5, 7, 8, 9, 10, 11, 12, 13, 14, 15, 16, 19, 20, 21 und 23 eine bessere antimykotische Wirkung als die aus dem Stand der Technik bekannten Verbindungen (A), (B), (C) und (D).

Tabelle A

Antimykotische in-vitro-Wirksamkeit

| Wirkstoff | MHK-Werte in /ml Nährmedium<br>Trichophyton mentagr. | Microsporum canis | Candida albicans | Torulopsis glabrata | Aspergillus fumigatus |
|---|---|---|---|---|---|
| (A) (bekannt) | 8 | 16 | 1 | 2 | 32 |
| (B) (bekannt) | 4 | 4 | 32 | >64 | 32 |
| (C) (bekannt) | 32 | - | 32 | >64 | >64 |
| (D) (bekannt) | 8 | 16 | 2 | 16 | 64 |
| Verbindungen gemäß Herst. Bsp. | | | | | |
| 3 | <1 | 8 | 8 | 8 | 8 |
| 5 | <1 | <1 | <1 | 8 | 4 |
| 7 | <1 | 2 | <1 | 2 | <1 |
| 8 | <1 | <1 | <1 | 8 | 2 |
| 9 | <1 | 4 | <1 | 8 | 4 |
| 10 | <1 | <1 | <1 | 4 | <1 |
| 11 | <2 | 2 | 2 | 16 | 16 |
| 12 | <1 | 16 | <1 | 32 | 8 |
| 13 | <1 | 16 | 2 | 32 | 32 |
| 14 | <1 | 8 | <1 | 2 | <1 |
| 15 | <1 | 8 | <1 | 4 | 8 |
| 16 | 2 | 16 | <1 | 32 | 32 |
| 19 | <1 | 4 | <1 | 2 | 4 |
| 20 | <1 | <1 | <1 | 2 | 16 |
| 21 | 4 | <1 | <1 | 16 | 32 |
| 23 | 2 | <1 | <1 | 8 | 32 |

## Beispiel B

Antimykotische in-vivo-Wirksamkeit (oral) bei Mäusecandidose

Versuchsbeschreibung

Mäuse vom Typ SPF-$CF_1$ wurden intravenös mit 1-2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50-100 mg/kg Körpergewicht der Präparate oral behandelt.

Ergebnis

Unbehandelte Tiere starben 3 bis 6 Tage post infektionem. Die Überlebensrate am 6. Tag post infektionem betrug bei unbehandelten Kontrolltieren etwa 5 %.

In diesem Test zeigten z. B. die erfindungsgemäßen Verbindungen 3, 7, 8, 9, 10, 14, 15, 16, 19, 20, 21, 22, 23 und 26 eine bessere Wirkung als die aus dem Stand der Technik bekannten Verbindungen (A), (B), (C) und (D).

Zeichenerklärung

+++++ = sehr gute Wirkung = 90 % Überlebende am 6. Tag p. i.
++++ = gute Wirkung = 80 % Überlebende am 6. Tag p. i.
+++ = Wirkung = 60 % Überlebende am 6. Tag p. i.
++ = schwache Wirkung = 40 % Überlebende am 6. Tag p. i.
\+ = Spur Wirkung = unter 40 % Überlebende am 6. Tag p. i.
k. W. = keine Wirkung

### Tabelle B

Antimykotische in-vivo-Wirkung (oral) bei Mäusecandidose

| Wirkstoff | Wirkung |
|---|---|
| (A) (bekannt) | k. W. |
| (B) (bekannt) | k. W. |
| (C) (bekannt) | k. W. |
| (D) (bekannt) | k. W. |
| Verbindungen gemäß den Herstellungsbeispielen | |
| 3 | +++ |
| 7 | ++++ |
| 8 | +++ |
| 9 | +++++ |
| 10 | +++ |
| 14 | ++++ |
| 15 | ++++ |
| 16 | ++++ |
| 19 | +++++ |
| 20 | ++++ |
| 21 | +++ |
| 22 | +++++ |
| 23 | +++++ |
| 26 | +++ |

## Beispiel C

Antimikrobielle in-vivo-Wirksamkeit (lokal) am Modell der experimentellen Meerschweinchen-Trichophytie

Versuchsbeschreibung

Weiße Mäuse der Rasse Pribright-white wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p. i., 1 × täglich mit einer 15 %igen Lösung der erfindungsgemäßen Präparate (in Dimethylsulfoxid : Glycerin = 1 : 4) local behandelt.

Ergebnis

Bei unbehandelten Tieren entwickelte sich innerhalb 12 Tagen p. i. das typische Bild einer Dermatophytose mit Rötung, Schuppung und Haarausfall bis zum totalen Integument-Defekt an der Infektionsstelle.

In diesem Test zeigten z. B. die erfindungsgemäßen Verbindungen 7, 8, 9, 10, 14, 19, 20, 21, 22 und 23 Wirkung bis gute Wirkung.

Tabelle C

Antimykotische in-vivo-Wirksamkeit (local) am Modell der experimentellen Meerschweinchen-Trichophytie

| Wirkstoff gemäß Herst. Beispiel | Wirkung |
|---|---|
| 7 | +++ |
| 8 | ++ |
| 9 | ++++ |
| 10 | ++++ |
| 14 | ++++ |
| 19 | +++ |
| 20 | ++ |
| 21 | ++ |
| 22 | +++ |
| 23 | ++ |

+++++ = sehr gute Wirkung = keine Infektionszeichen am 12. bis 15. Tag p. i.
++++ = gute Wirkung = geringe Rötung, vereinzelt Schuppen
+++ = Wirkung = Rötung, Schuppen, ohne Haarausfall
++ = schwache Wirkung = Rötung, Schuppung, Haarausfall
+ = Spur Wirkung = flächiger Haarausfall, entzündliche Hautreaktion

Beispiel D/Formulierungen

1. Lösung

| | |
|---|---|
| Wirkstoff gemäß Formel (I) : | 10 g |
| Alkohol, rein (96 %ig) : | 300 g |
| Isopropylmyristat : | 526 g |
| | 836 g |

2. Creme

| | |
|---|---|
| Wirkstoff gemäß Formel (I) : | 10 g |
| Arlacel 60 : | 20 g |
| (Sorbitan-monostearat) Twenn 60 : | 15 g |
| (Polyoxyethylen (20)-sorbitan-monostearat) Walrat, künstlich : | 30 g |
| (Mischung von Estern von gesättigten Fettsäuren $C_{14}$-$C_{18}$ und Fettalkoholen $C_{14}$-$C_{18}$) Lanette 0 : | 100 g |
| (Gemisch aus Cetyl-Alkohol und Stearyl-Alkohol) Ethanol G : | 135 g |
| (2-Octyl-dodecanol) Benzylalkohol : | 10 g |
| Wasser, entmineralisiert : | 680 g |
| | 1 000 g |

**Patentansprüche**

1. Heterocyclisch substituierte Hydroxyalkyl-azolyl-Derivate der allgemeinen Formel

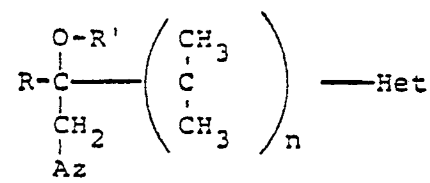

(I)

in welcher

Az für 1,2,4-Triazol-1-yl, 1,2,4-Triazol-4-yl oder Imidazol-1-yl steht,

Het für jeweils gegebenenfalls einfach oder mehrfach, gleich oder verschieden substituiertes Dioxolan-2-yl oder 1,3-Dioxanyl steht, wobei als Substituenten zu nennen sind : Alkyl mit 1 bis 4 Kohlenstoffatomen, sowie jeweils gegebenenfalls einfach oder mehrfach, gleich oder verschieden substituiertes Phenyl und Phenoxyalkyl mit 1 bis 4 Kohlenstoffatomen im Alkylteil, wobei als Phenylsubstituenten zu nennen sind : Halogen, Alkyl mit 1 bis 4 Kohlenstoffatomen, Alkoxy und Alkylthio mit jeweils 1 bis 2 Kohlenstoffatomen sowie halogenalkyl, Halogenalkoxy und Halogenalkylthio mit jeweils 1 bis 2 Kohlenstoffatomen und 1 bis 5 gleichen oder verschiedenen Halogenatomen ;

R für geradkettiges oder verzweigtes Alkyl mit 1 bis 7 Kohlenstoffatomen steht, ferner für jeweils gegebenenfalls einfach oder mehrfach, gleich oder verschieden substituiertes Phenyl, Phenylalkyl mit 1 bis 4 Kohlenstoffatomen im Alkylteil, Phenoxy- und Phenylthioalkyl mit jeweils 1 bis 4 Kohlenstoffatomen im Alkylteil oder für Phenylethenyl steht, wobei als Substituenten an den Phenyl-Gruppen genannt werden : Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und Alkyl mit 1 bis 2 Kohlenstoffatomen substituiertes Phenyl, Phenoxy, Benzyl und Benzyloxy ; weiterhin für jeweils gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen steht, für Cycloalkylmethyl oder -ethyl mit 5 bis 7 Kohlenstoffatomen im Cycloalkylteil und Cyclohexylethenyl steht, schließlich für Alkenyl mit 2 bis 6 Kohlenstoffatomen, für 1,2,4-Triazol-1-yl-methyl, 1,2,4-Triazol-4-yl-methyl, Imidazol-1-yl-methyl und Pyrazol-1-yl-methyl steht,

R' für Wasserstoff, für gegebenenfalls durch Phenyl substituiertes Alkyl mit 1 bis 4 Kohlenstoffatomen, wobei der Phenylrest durch die bei R genannten Phenylsubstituenten substituiert sein kann, und für Alkenyl mit 2 bis 4 Kohlenstoffatomen steht ; und

n für die Zahlen 0 oder 1 steht,

wobei für den Fall, daß n für 0 steht, Het ein gegebenenfalls wie oben ausgeführt substituiertes 1,3-Dioxan-5-yl sein muß,

und deren Säureadditionssalze und Metallsalz-Komplexe zur Bekämpfung von Mykosen.

2. Verbindungen der allgemeinen Formel (I) in Anspruch 1, wobei

Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten zu nennen sind : Methyl, Ethyl, n-Propyl, Isopropyl sowie gegebenenfalls einfach bis dreifach, gleich oder verschieden durch Fluor, Chlor, Methyl, Trifluormethyl oder Trifluormethoxy substituiertes Phenyl und Phenoxymethyl ;

R für tert.-Butyl, Trimethyl-propyl, Tetramethylpropyl sowie für jeweils gegebenenfalls einfach bis dreifach, gleich oder verschieden substituiertes Phenyl, Benzyl, Phenethyl, Phenoxythenyl, Phenylthiomethyl oder Phenethenyl steht, wobei als Phenylsubstituenten zu nennen sind : Fluor, Chlor, Methyl, Trifluormethyl, Trifluormethoxy, Trifluormethylthio, Hydroxyiminomethyl, 1-Hydroximinoethyl, Methoximinomethyl und 1-Methoximinoethyl sowie jeweils gegebenenfalls durch Chlor und/oder Methyl substituiertes Phenyl, Phenoxy, Benzyl und Benzyloxy ; R ferner für jeweils gegebenenfalls einfach bis zweifach, gleich oder verschieden durch Methyl, Ethyl oder Isopropyl substituiertes Cyclohexyl, Cyclohexylmethyl, Cyclohexylethyl oder Cyclohexylethenyl steht ; R weiterhin für Allyl, Dimethylpropenyl, 1,2,4-Triazol-1-yl-methyl, 1,2,4-Triazol-4-yl-methyl, Imidazol-1-yl-methyl oder Pyrazol-1-yl-methyl steht ;

R' für Wasserstoff, Methyl, 4-Chlorbenzyl oder Allyl steht, und

Az und n die in Anspruch 1 angegebene Bedeutung besitzen,

wobei für den Fall, daß n für 0 steht, Het ein gegebenenfalls wie oben ausgeführt substituiertes 1,3-Dioxan-5-yl sein muß,

und deren Säureadditionssalze und Metallsalz-Komplexe zur Bekämpfung von Mykosen.

3. Antimykotische Mittel, gekennzeichnet durch einen Gehalt an mindestens einem heterocyclisch substituierten Hydroxyalkyl-azolyl-Derivat der Formel (I) in Anspruch 1.

4. Verfahren zur Herstellung von antimykotischen Mitteln, dadurch gekennzeichnet, daß man heterocyclisch substituierte Hydroxyalkyl-azolyl-Derivate der Formel (I) in Anspruch 1 mit inerten, nichttoxischen, pharmazeutisch geeigneten Trägerstoffen vermischt.

## Claims

1. Heterocyclically substituted hydroxyalkyl-azolyl derivatives of the general formula

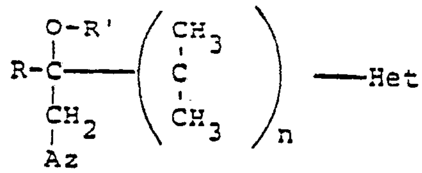

(I)

in which

Az represents 1,2,4-Triazol-1-yl, 1,2,4-triazol-4-yl or imidazol-1-yl,

Het represents dioxolan-2-yl or 1,3-dioxanyl, each of which is optionally mono- or polysubstituted by identical or different substituents, the substituents to be mentioned being : alkyl with 1 to 4 carbon atoms, and phenyl and phenoxyalkyl with 1 to 4 carbon atoms in the alkyl part, each of which is optionally mono- or polysubstituted by identical or different substituents, the phenyl substituents to be mentioned being : halogen, alkyl with 1 to 4 carbon atoms, alkoxy and alkylthio, each with 1 to 2 carbon atoms, and halogenoalkyl, halogenoalkoxy and halogenoalkylthio, each with 1 to 2 carbon atoms and 1 to 5 identical or different halogen atoms ;

R represents straight-chain or branched alkyl with 1 to 7 carbon atoms, also phenyl, phenylalkyl with 1 to 4 carbon atoms in the alkyl part, phenoxy- and phenylthioalkyl, each with 1 to 4 carbon atoms in the alkyl part, each of which is optionally mono- or polysubstituted by identical or different substituents, or represents phenylethenyl, the following being mentioned as substituents on the phenyl groups : halogen, alkyl with 1 to 4 carbon atoms, alkoxy and alkylthio, each with 1 to 2 carbon atoms, nitro, halogenoalkyl and halogenalkoxy and halogenoalkylthio, each with 1 to 2 carbon atoms and 1 to 5 identical or different halogen atoms, hydroximinoalkyl with 1 to 4 carbon atoms, alkoximinoalkyl with 1 to 4 carbon atoms in each alkyl part, and phenyl, phenoxy, benzyl and benzyloxy, each of which is optionally substituted by halogen and alkyl with 1 to 2 carbon atoms ; also represents cycloalkyl which has 5 to 7 carbon atoms and is in each case optionally mono- or polysubstituted by identical or different alkyl radical with 1 to 4 carbon atoms, and represents cycloalkylmethyl or -ethyl with 5 to 7 carbon atoms in the cycloalkyl part and cyclohexylethenyl, and finally represents alkenyl with 2 to 6 carbon atoms, 1,2,4-triazol-1-yl-methyl, 1,2,4-triazol-4-yl-methyl, imidazol-1-yl-methyl and pyrazol-1-yl-methyl,

R′ represents hydrogen, optionally phenyl-substituted alkyl with 1 to 4 carbon atoms, it being possible for the phenyl radical to be substituted by the phenyl substituents mentioned under R, and represents alkenyl with 2 to 4 carbon atoms ; and

n represents the numbers 0 or 1,

wherein in the case where n represents 0, Het must be a 1,3-dioxan-5-yl optionally substituted as described above and their acid addition salts and metal salt complexes for combating mycoses.

2. Compounds of the general formula (I) in Claim 1, wherein

Het represents dioxolan-2-yl, 1,3-dioxan-5-yl or 1,3-dioxan-2-yl, each of which is optionally mono- to trisubstituted by identical or different substituents, the substituents to be mentioned being : methyl, ethyl, n-propyl, isopropyl, and phenyl and phenoxymethyl, optionally mono- to trisubstituted by identical or different substituents from the group comprising fluorine, chlorine, methyl, trifluoromethyl and trifluoromethoxy ;

R represents tert.-butyl, trimethyl-propyl, tetramethyl-propyl, and phenyl, benzyl, phenethyl, phenoxymethyl, phenylthiomethyl or phenethenyl, each of which is optionally mono- to trisubstituted by identical or different substituents, the phenyl substituents to be mentioned being : fluorine, chlorine, methyl, trifluoromethyl, trifluoromethoxy, trifluoromethylthio, hydroxyiminomethyl, 1-hydroximinoethyl, methoximinomethyl and 1-methoximinoethyl and phenyl, phenoxy, benzyl and benzyloxy, each of which is optionally substituted by chlorine and/or methyl ; R furthermore represents cyclohexyl, cyclohexylmethyl, cyclohexylethyl or cyclohexylethenyl, each of which is optionally mono- to disubstituted by identical or different substituents from the group comprising methyl, ethyl and isopropyl ; R furthermore represents allyl, dimethylpropenyl, 1,2,4-triazol-1-yl-methyl, 1,2,4-triazol-4-yl-methyl, imidazol-1-yl-methyl or pyrazol-1-yl-methyl ;

R′ represents hydrogen, methyl, 4-chlorobenzyl or allyl, and

Az and n have the meaning given in Claim 1, wherein in the case where n represents 0, Het must be a 1,3-dioxan-5-yl optionally substituted as described above,

and their acid addition salts and metal complexes, for combating mycoses.

3. Antimycotic agents, characterised in that they contain at least one heterocyclically substituted hydroxyalkyl-azolyl derivative of the formula (I) in Claim 1.

4. Process for the preparation of antimycotic agents, characterised in that heterocyclically substituted hydroxyalkylazolyl derivatives of the formula (I) in Claim 1 are mixed with inert, non-toxic, pharmaceutically suitable excipients.

**Revendications**

1. Dérivés hydroxyalkyl-azolyliques à substituant hétérocyclique, de formule générale

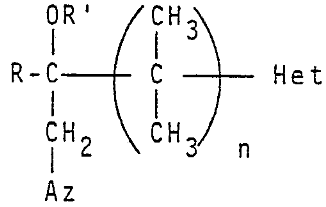

(I)

dans laquelle

Az représente un groupe 1,2,4-triazole-1-yle, 1,2,4-triazole-4-yle ou imidazole-1-yle,

Het représente un groupe dioxolanne-2-yle et 1,3-dioxannyle éventuellement substitué une ou plusieurs fois, de façon identique ou différente, et l'on peut citer comme substituants : un groupe alkyle ayant 1 à 4 atomes de carbone, ainsi qu'un groupe phényle et phénoxyalkyle ayant 1 à 4 atomes de carbone dans la partie alkyle, éventuellement substitués une fois ou plusieurs fois, de façon identique ou différente, et l'on peut citer comme substituants du groupe phényle : un halogène, un groupe alkyle ayant 1 à 4 atomes de carbone, un groupe alcoxy et alkylthio ayant chacun 1 ou 2 atomes de carbone ainsi qu'un groupe halogénoalkyle, halogénoalcoxy et halogénoalkylthio ayant chacun 1 ou 2 atomes de carbone et 1 à 5 atomes d'halogène identiques ou différents ;

R représente un groupe alkyle linéaire ou ramifié ayant 1 à 7 atomes de carbone, ainsi qu'un groupe phényle, phénylalkyle ayant 1 à 4 atomes de carbone dans la partie alkyle, phénoxyalkyle et phénylthioalkyle ayant chacun 1 à 4 atomes dans la partie alkyle ou phénéthényle (chacun étant éventuellement substitué une ou plusieurs fois, de façon identique ou différente), et on peut citer comme substituants des groupes phényles : 1 atome d'halogène, un groupe alkyle ayant 1 à 4 atomes de carbone, un groupe alcoxy et alkylthio ayant chacun 1 ou 2 atomes de carbone, un groupe nitro, halogénoalkyle et halogénoalcoxy ainsi que halogénoalkylthio ayant chacun 1 ou 2 atomes de carbone et 1 à 5 atomes d'halogène identique ou différent, un groupe hydroximinoalkyle ayant 1 à 4 atomes de carbone, alcoximinoalkyle ayant 1 à 4 atomes de carbone dans chaque partie alkyle, ainsi qu'un groupe phényle, phénoxy, benzyle et benzyloxy (chacun éventuellement substitué par de l'halogène et par un groupe alkyle ayant 1 ou 2 atomes de carbone) ; ainsi qu'un groupe cycloalkyle ayant 5 à 7 atomes de carbone, éventuellement substitué une ou plusieurs fois de façon identique ou différente par un groupe alkyle ayant 1 à 4 atomes de carbone, un groupe cycloalkylméthyle ou cycloalkyléthyle ayant 5 à 16 atomes de carbone dans la partie cycloalkyle et cyclohexyléthényle, enfin un groupe alcényle ayant 2 à 6 atomes de carbone, un groupe 1,2,4-triazole-1-yl-méthyle, 1,2,4-triazole-4-yl-méthyle, imidazole-1-yl-méthyle et pyrazol-1-yl-méthyle,

R' représente un atome d'hydrogène, un groupe alkyle ayant 1 à 4 atomes de carbone et éventuellement substitué par un reste phényle, le reste phényle pouvant être substitué par les substituants du reste phényle cités pour R, et R' peut représenter un groupe alcényle ayant 2 à 4 atomes de carbone ; et

n représente les nombres 0 ou 1 et,

si n est nul, Het doit représenter un groupe 1,3-dioxanne-5-yle éventuellement substitué comme indiqué ci-dessus

et leurs sels d'addition d'acide et leurs complexes de sels de métaux, pour combattre les mycoses.

2. Composés de formule générale (I) dans la revendication 1, dans laquelle

Het représente un groupe dioxolanne-2-yle, 1,3-dioxanne-5-yle ou 1,3-dioxanne-2-yle (éventuellement substitué une à trois fois, de façon identique ou différente), et l'on peut citer comme substituants : un groupe méthyle, éthyle, n-propyle, isopropyle ; ainsi qu'un groupe phényle et phénoxyméthyle (éventuellement substitué une à trois fois, de façon identique ou différente, par un atome de fluor ou de chlore, un groupe méthyle, trifluorométhyle ou trifluorométhoxy) ;

R représente un groupe tertiobutyle, triméthyl-propyle, tétraméthylpropyle, ainsi qu'un groupe phényle, benzyle, phénéthyle, phénoxyméthyle, phénylthiométhyle ou phénéthényle (éventuellement substitués chacun une à trois fois, de façon identique ou différente), et l'on peut citer comme substituants du groupe phényle : un atome de fluor ou de chlore, un groupe méthyle, trifluorométhyle, trifluorométhoxy, trifluorométhylthio, hydroximinométhyle, 1-hydroximinoéthyle, méthoximinométhyle et 1-méthoximinoéthyle ; ainsi qu'un groupe phényle, phénoxy, benzyle et benzyloxy (éventuellement substitués chacun par du chlore et/ou par un groupe méthyle) ; R représente en outre un groupe cyclohexyle, cyclohexylméthyle, cyclohexyléthyle ou cyclohexyléthényle (éventuellement substitués chacun, une à deux fois, de façon identique ou différente, par un reste méthyle, éthyle ou isopropyle) ; R représente en outre un groupe allyle, diméthylpropényle, 1,2,4-triazole-1-yl-méthyle, 1,2,4-triazole-4-yl-méthyle, imidazole-1-yl-méthyle ou pyrazol-1-yl-méthyle ;

R' représente un atome d'hydrogène, un groupe méthyle, 4-chlorobenzyle ou allyle ; et

Az et n ont le sens indiqué à la revendication 1, et, si n est nul, Het doit représenter un groupe 1,3-dioxanne-5-yle éventuellement substitué comme indiqué ci-dessus, et leurs sels d'addition d'acides et complexes de sels de métaux, pour combattre des mycoses.

3. Produit antimycotique, caractérisé en ce qu'il contient au moins un dérivé hydroxyalkyl-azolylique à substituant hétérocyclique de formule (I) de la revendication 1.

4. Procédé pour fabriquer des produits antimycotiques, caractérisé en ce qu'on mélange des dérivés hydroxyalkyl-azolyliques à substituant hétérocyclique, de formule (I) de la revendication 1, avec des excipients pharmaceutiquement convenables, inertes, non toxiques.